# EUROPEAN PATENT APPLICATION

(11) **EP 4 474 879 A1**
(43) Date of publication of application: **11.12.2024**
(21) Application number: 23177608.9
(22) Date of filing: 06.06.2023
(51) Int. Cl.: G02B 7/00, G02B 21/00

(54) **APPARATUS FOR AN OPTICAL IMAGING SYSTEM, OPTICAL IMAGING SYSTEM, METHOD AND COMPUTER PROGRAM**

(71) Applicant: Leica Instruments (Singapore) Pte Ltd, Singapore 608924 (SG)
(72) Inventor: THEMELIS, George, 88131 Lindau (DE)
(74) Representative: 2SPL Patentanwälte PartG mbB

(57) **Abstract**

Examples relate to an apparatus for an optical imaging system, comprising one or more processors and one or more storage devices. The apparatus is configured to determine axis data indicative of a visual representation of an axis for arranging a microscope of the optical imaging system. Further, the apparatus is configured to transmit the axis data and obtain movement data indicative of a movement of a hand of the user of the optical imaging system relative to the visual representation of the axis. Further, the apparatus is configured to determine control data for arranging the microscope based on the movement data.

## Description

### Technical field

Examples relate to an apparatus for an optical imaging system, such as surgical optical imaging system, and an optical imaging system, a method, and a computer program.

### Background

Conventional analog microscopes require a user to be in physical contact with the microscope and employ handles that allow the user to align the microscope. For example, an exoscope is designed for heads-up use, i.e., the user is looking at a screen and the microscope is out of the way, and the user does not need to have physical contact to the exoscope. However, even for an exoscope the alignment is still done by physically grabbing and repositioning of the microscope.

Thus, there may be a desire for an improved concept for aligning a microscope.

### Summary

This desire is addressed by the subject-matter of the independent claims.

The concept proposed in the present disclosure is based on the insight, that an alignment or arrangement of a microscope, e.g., an exoscope, can be controlled based on control data. The control data may be indicative of an arrangement of the microscope. The control data can be determined based on the movement of a hand of the user relative to a visual presentation of an axis. In this way, the user can use the visual presentation of the axis for arranging the microscope by a movement of his hand. Therefore, the arrangement of the microscope can be facilitated and/or a user experience can be improved. A physical contact with the microscope can be avoided.

Examples provide an apparatus for an optical imaging system, comprising one or more processors and one or more storage devices. The apparatus is configured to determine axis data indicative of a visual representation of an axis for arranging a microscope of the optical imaging system. Further, the apparatus is configured to transmit the axis data and obtain movement data indicative of a movement of a hand of the user of the optical imaging system relative to the visual representation of the axis. Further, the apparatus is configured to determine control data for arranging the microscope based on the movement data. The control data may be indicative of a movement of the microscope, e.g., of a movement of the microscope relative to a sample, a movement of an objective of the microscope. For example, the control data can be used to trigger a movement of the microscope. Thus, the microscope can be arranged based on the movement data. Determining and transmitting the axis data may allow to trigger display of the visual representation of the axis to a user. For example, the axis data may be transmitted to a display device and the visual presentation of the axis can be displayed on the display device, e.g., a head mounted display. Thus, the apparatus can provide information to the user about an axis for arranging the microscope. For example, the axis may be a visual handle for arranging the microscope. Thus, the user can use the axis to arrange the microscope. The movement data may allow to determine an intention of the user to move the microscope, e.g., for arranging the microscope. Thus, by obtaining the movement data the apparatus can determine the control data based on the movement data. Therefore, the control data may be indicative of a movement of the microscope desired by the user of the optical imaging system. In this way, an arrangement of the microscope can be performed by use of the control data. Thus, the apparatus can trigger and/or control the arrangement of the microscope based on the control data.

In an example, the apparatus may be further configured to determine gesture data indicative of a gesture being performed by the user. The gesture data is determined based on the movement data. Further, the apparatus may be configured to determine the control data based on the movement data and the gesture data. By determining the gesture data a reliability of the control data can be increased. For example, only a predefined gesture performed by the user may be indicative for a desire of the user to adjust an arrangement of the microscope. Thus, movement of the hand of the user not intended to arrange the microscope can be neglected. In this way, a user experience can be increased.

In an example, the apparatus may be further configured to control an observation angle of the microscope, a magnification of the microscope and/or a field of view of the microscope. The control is based on the control data. In this way, the user can arrange the microscope in a desired way. The arrangement of the microscope may comprise an adjustment of parameters of the microscope, such like a magnification, a field of view and/or an observation angle.

In an example, the visual representation of the axis may be a visual presentation of the optical path of the microscope or a handle of the microscope. Thus, an arrangement of the microscope can be performed by the user with respect to a desired fixed point. For example, if the visual representation of the axis is a visual representation of an optical path of the microscope, the user can adjust an arrangement of the microscope by virtually moving the microscope. For example, if the visual presentation of the axis is a handle of the microscope, the user can adjust an arrangement of the microscope by moving an imaged sample, i.e., an image of the sample acquired by the microscope and displayed on a display device. In this way, the user can decide whether he wants to arrange the microscope by virtually moving the microscope (moving the hand in the same direction as the change of the observation angle) or using a virtual handle (moving the hand in the opposite direction as the change of the observation angle).

In an example, the apparatus may be further configured to determine temporary data indicative of a temporary view of the sample for displaying during arrangement of the microscope and transmit the temporary data. For example, the temporary data may be transmitted to the display device or a frame buffer. Thus, the display device can display the temporary data during arrangement of the microscope. In this way, the user can see the temporary view of the sample during the arrangement of the microscope. Therefore, a view of the sample can be provided to the user during arrangement of the microscope, which may improve a user experience.

In an example, the apparatus may be further configured to receive sensor data of a sensor of the optical imaging system. The sensor data is indicative of a life view of the sample through the microscope of the optical imaging system. Further, the apparatus may be configured to determine the temporary data indicative of an extrapolated view of the sample. The temporary data may be determined based on the sensor data and the control data. Using the control data the temporary data can be determined for a time of the arrangement of the microscope. For example, the control data may be indicative of a time needed to arrange the microscope. Further, using sensor data the temporary data can be improved, such that the temporary view of the sample during arrangement of the microscope has an increased reliability.

In an example, the apparatus may be further configured to obtain reference point data indicative of a reference point for the arrangement of the microscope and determine the control data based on the movement data and the reference point data. The reference point may be a point relative to which the microscope is moved. For example, the user can select a rotation point around which the microscope is rotated relative to the sample. Thus, a user experience can be increased.

In an example, the apparatus may be further configured to receive movement sensor data of a movement sensor. The movement sensor data is indicative of a movement of the hand of the user. Further, the apparatus may be configured to determine the control data based on the movement sensor data. In this way, the movement data can be obtained in a facilitated way.

In an example, the apparatus may be further configured to obtain verification data indicative of an intention of the user to arrange the microscope and determine the control data based on the movement data and the verification data. The intention of the user to arrange the microscope can be verified based on the verification data. In this way, a reliability of the arrangement of the microscope can be increased.

In an example, the apparatus may be further configured to determine, based on the verification data, an intention of the user to adjust an observation angle of the microscope, a magnification of the microscope and/or a field of view of the microscope. Thus, the user can adjust a desired parameter of the microscope in a facilitated way.

In an example, the apparatus may be further configured to receive verification sensor data of a verification sensor. The verification sensor data is indicative of an intention of the user to arrange the microscope. The verification sensor is different from the movement sensor. Further, the apparatus may be configured to determine the verification data based on the verification sensor data. Using two different sensor to receive the verification sensor data and the movement sensor data may increase a reliability of the determination of the intention of the user.

In an example, the verification sensor may be a microphone. Thus, the user can easily select a parameter of the microscope to be adjusted by voice.

In an example, the apparatus may be further configured to obtain setting data indicative of a desire of the user to change the arrangement of the microscope. Further, the apparatus may be configured to determine the control data based on the movement data and the setting data. For example, the setting data may be received from an input device. In this way, the user can easily select a parameter of the microscope to be adjusted.

Examples provide an optical imaging system comprising an apparatus as described above.

Examples provide a method comprising determining axis data indicative of a visual representation of an axis for arranging a microscope of the optical imaging system. Further, the method comprises transmitting the axis data and obtaining movement data indicative of a movement of a hand of a user of the optical imaging system relative to the visual representation of the axis. The method further comprises determining control data for arranging the microscope based on the movement data.

Various examples of the present disclosure relate to a corresponding computer program with a program code for performing the above method when the computer program is executed on a processor.

### Short description of the Figures

Some examples of apparatuses and/or methods will be described in the following by way of example only, and with reference to the accompanying figures, in which
Figs. 1 and shows a schematic diagram of an example of an apparatus for an optical imaging system;
Figs. 2a-2d show different examples of an arrangement of the microscope using a visual representation of an axis;
Fig. 3 shows a schematic illustration of a system; and
Fig. 4 shows a flow chart of an example of a method for an optical imaging system.

### Detailed Description

Various examples will now be described more fully with reference to the accompanying drawings in which some examples are illustrated. In the figures, the thicknesses of lines, layers and/or regions may be exaggerated for clarity.

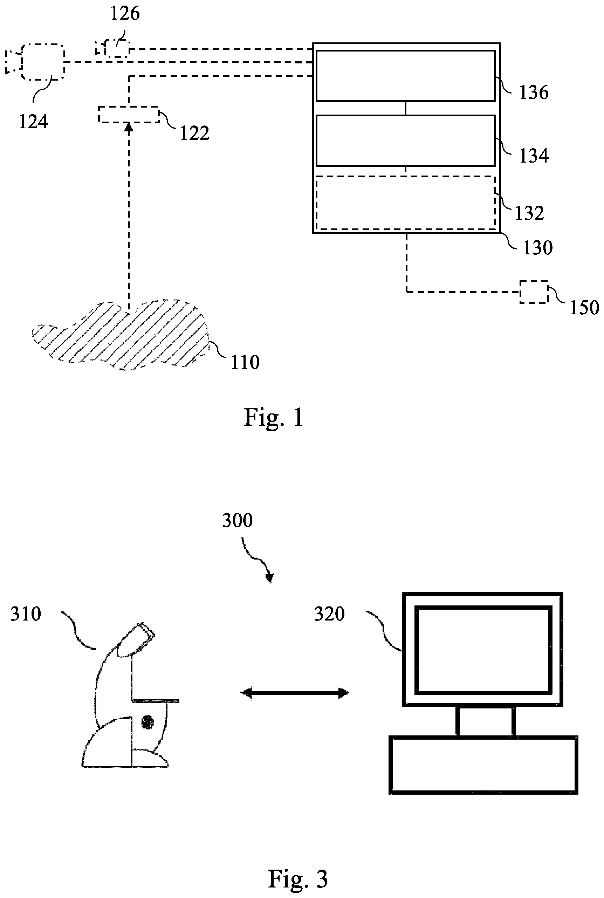

Figs. 1 shows a schematic diagram of an example of an apparatus 130 for an optical imaging system. The apparatus 130 is tasked with controlling various aspects of a microscope of the optical imaging system, which may be a surgical optical imaging system, and of the entire optical imaging system and/or with processing various types of sensor data of the optical imaging system. Consequently, the apparatus 130 may be implemented as a computer system, which interfaces with the various components of the optical imaging system, e.g., the sensor 122.

The apparatus 130 comprises, as shown in Fig. 1, one or more processors 134 and one or more storage devices 136. Optionally, the apparatus 130 further comprises one or more interfaces 132. The one or more processors 134 are coupled to the one or more storage devices 136 and to the optional one or more interfaces 132. In general, the functionality of the apparatus 130 may be provided by the one or more processors 134 (for determining axis data and/or control data), in conjunction with the one or more interfaces 132 (for exchanging information, e.g., with the sensor 122, a movement sensor 124 and/or a verification sensor 126) and/or with the one or more storage devices 136 (for storing and/or retrieving information).

The apparatus 130 is configured to determine axis data indicative of a visual representation of an axis for arranging a microscope of the optical imaging system. The axis data can be used to arrange the microscope. For example, an arrangement of the microscope may be a movement of the microscope relative to the sample 110. For example, the sensor 122 may be part of the microscope. Thus, by moving the sensor 122 relative to the sample the microscope can be arranged. For example, an arrangement of the microscope relative to the sample can be adjusted by moving the microscope and/or the sample 110.

Moving the microscope relative to the sample may allow to adjust an observation angle of the microscope. The observation angle of a microscope refers to the angle at which the microscope's objective lens or lenses are positioned relative to the sample 110 being observed. It determines the direction and path of light entering the microscope and interacting with the sample 110. The observation angle affects the resolution, contrast, and depth perception of the observed image.

Optionally or alternatively, arranging the microscope may comprise adjusting a parameter of the microscope such like a magnification, a field of view. Thus, an arrangement of the microscope can be performed without moving an outer part, e.g., a housing, of the microscope relative to the sample. For example, an arrangement of the microscope may comprise any action related to the microscope to adjust an image of the sample 110 acquired by the microscope e.g., a magnification, a focus, the field of view, an observation angle. In this way, the virtual representation of the axis can be used to adjust each parameter of the microscope relevant for image acquisition of the sample 110.

To inform the user about the visual representation of the axis the apparatus 130 is further configured to transmit the axis data. The axis data can be transmitted to a storage circuitry, such like a frame buffer, and/or a display device, such like a head mounted display device. For example, the axis data can be temporarily stored in the frame buffer and transmitted from the frame buffer to the display device. The display device can be used to display the visual representation of the axis to the user. Thus, the user can be informed about the axis and can perform certain measures to interact with the axis.

For example, the user may use the axis to control the movement of the microscope relative to the sample 110. Therefore, the apparatus 130 is further configured to obtain movement data indicative of a movement of a hand of the user of the optical imaging system relative to the visual representation of the axis. The movement data can be received from a movement sensor 124. Alternatively, the movement data can be measured by the movement sensor 124. In this case, the movement sensor 124 may be part of the apparatus 130.

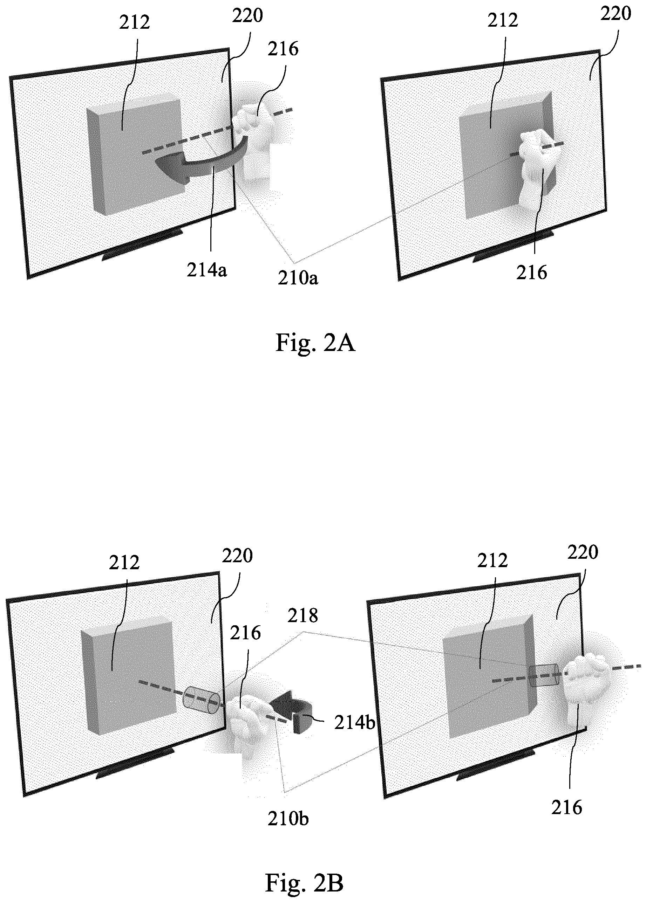

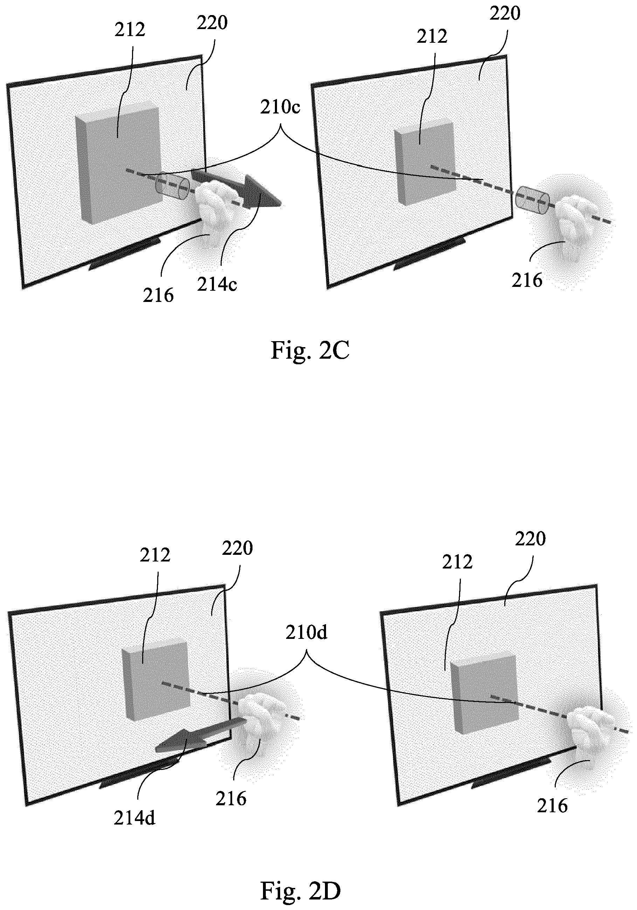

Obtaining the movement data may allow to use contactless control, such like finger-pointing, hand waving, hand swiping, palm gesture, for easy intuitive and/or fast alignment of the microscope. Contactless control is enabled by the visual representation of the axis. The axis can visualize a virtual handle that the user can interact with. For example, the user can use the virtual handle and can adjust an observation angle of the microscope (see Fig. 2A and 2B), a magnification (see Fig 2C) and/or field of view (see Fig. 2D). The user experience may be based on a virtual reality comprising the visual representation of the axis. Optionally the user experience may be based on computer aided design for illustrating an impact of the arrangement of the microscope (see Fig. 2).

To trigger or control the arrangement of the microscope the apparatus 130 is further configured to determine control data for arranging the microscope based on the movement data. The control data may be indicative of a movement of the microscope relative to the sample 110. For example, the movement data may be indicative of a movement of the microscope and/or of a movement of the sample 110. A movement of the microscope and/or the sample can be a translational and/or a rotational movement, for example. For example, the movement of the microscope can be a movement of an objective, e.g., to adjust a focus and/or a magnification (e.g., by changing an objective). In this way, the user can adjust a desired parameter, especially each parameter, of the microscope associated with image acquisition in a facilitated way.

For example, the apparatus 130 can trigger the arrangement of the microscope by transmitting the control data. The control data can be transmitted to an actuator of the optical imaging system, e.g., the microscope, and/or to a processing circuitry of the optical imaging system, e.g., the microscope. Alternatively, the apparatus 130 may control the arrangement of the microscope by controlling the movement of the microscope based on the control data. For example, an actuator to adjust a position of the microscope may be part of the apparatus 130. For example, the apparatus 130 may be part of the microscope. Thus, the apparatus 130 can control and/or trigger the arrangement of the microscope.

By determining the control data the microscope can be arranged by the user without a need to physically grab a handle. Thus, a physical contact with the microscope can be omitted. In this way, an arrangement of the microscope can be improved.

The apparatus 130 may allow fast and intuitive control of the arrangement of the microscope. The user does not need to use any additional equipment such as handheld devices or wearing eye-trackers. The perceived response of the apparatus 130 can reach very low latency. Further, the apparatus 130 can support multiple users, e.g., a surgeon assistant could also perform the arrangement of the microscope when the surgeon does not have free hands.

The proposed concept may be built around two main components - the microscope, which comprises the optical components and the apparatus 130, which may be used to control the optical imaging system, process sensor data of the microscope 120, e.g., the sensor 122, and/or to determine the control data. In an example, an optical imaging system may comprise the apparatus 130.

In general, a microscope is an optical instrument that is suitable for examining objects that are too small to be examined by the human eye (alone). For example, a microscope may provide an optical magnification of a sample, such as a sample 110 shown in Fig. 1. In modern microscopes, the optical magnification is often provided for a camera or an imaging sensor, such as the optical imaging sensors 122 of the microscope. The microscope may further comprise one or more optical magnification components that are used to magnify a view of the sample 110, such as an objective.

There are a variety of different types of optical imaging systems. If the optical imaging system is used in the medical or biological fields, the sample may be a sample of organic tissue, e.g., arranged within a petri dish or present in a part of a body of a patient. In some examples of the present disclosure the optical imaging system may be a surgical optical imaging system, e.g., an optical imaging system that is to be used during a surgical procedure, such as an oncological surgical procedure or during tumor surgery. However, the proposed concept may also be applied to other types of microscopy, e.g., microscopy in a laboratory or microscopy for the purpose of material inspection.

In an example, the apparatus 130 may be further configured to determine gesture data indicative of a gesture being performed by the user. Further, the apparatus 130 may be configured to determine the control data based on the movement data and the gesture data. The gesture data is determined based on the movement data. For example, the user may perform a movement with his hand according to a predefined gesture, e.g., grab the axis, swipe his hand along the axis. Determining the gesture data may allow to improve a reliability of the control data. For example, the gesture of the user can be easily identified by the apparatus 130. Thus, a determination of an intention of the user to arrange the microscope can be improved. Further, movement data not indicative of a gesture of the hand of the user can be discarded. Thus, the determination of the control data can be improved.

Optionally, the gesture data can be used as starting point for determining a desired arrangement of the microscope. For example, only the movement of the hand of the user after the gesture may be used by the apparatus 130 to determine the arrangement of the microscope.

Optionally, the gesture data can be used to define a desired arrangement of the microscope. For example, a first gesture, such like grabbing the axis, may indicate a desire of the user to change an observation angle of the microscope and a second gesture, such like swiping the hand along the axis, may indicate a desire of the user to change the magnification of the microscope. In this way, an arrangement of the microscope can be improved using the gesture data.

In an example, the apparatus 130 may be further configured to control or trigger an observation angle of the microscope, a magnification of the microscope and/or a field of view of the microscope. The control or trigger is based on the control data. Therefore, a user can easily adjust different desired parameters of the microscope for image acquisition. Optionally, the user can adjust each parameter of the microscope relevant to image acquisition of the sample 110.

In an example, the visual representation of the axis may be a visual presentation of the optical path of the microscope or a handle of the microscope. For example, the user can either rotate an image of the sample displayed on the display device (see Fig. 2a) or virtually move the microscope (see Fig. 2b). Thus, the user can adjust the arrangement of the microscope in a desired way.

In an example, the apparatus 130 may be further configured to determine temporary data indicative of a temporary view of the sample 110 for displaying during arrangement of the microscope and transmit the temporary data. During the arrangement of the microscope, i.e., during the movement of the microscope relative to the sample 110 and/or of the objective of the microscope, no image acquisition of the sample 110 may be possible. Thus, no currently acquired image of the sample 110 can be displayed to the user. Therefore, the apparatus 130 may determine temporary data to trigger and/or control displaying a temporary view of the sample 110 on the display device. In this way, the apparatus 130 can permanently provide a view of the sample 110 to the user. Thus, the user experience can be increased.

In an example, the apparatus 130 may be further configured to receive sensor data of a sensor 122 of the optical imaging system. The sensor data is indicative of a life view of the sample 110 through the microscope of the optical imaging system. Further, the apparatus 130 may be configured to determine the temporary data indicative of an extrapolated view of the sample 110. The temporary data may be determined based on the sensor data and the control data. For example, the control data may be indicative of a time required to arrange the microscope. For example, moving the microscope relative to the sample 110 may take a time depending on the movement speed of the microscope. Thus, based on the control data, a time can be determined for which no image acquisition of the sample 110 is possible. In this way, the apparatus 130 can determine the extrapolated view for the time for which no image acquisition of the sample 110 is possible.

The sensor data may allow the apparatus 110 to determine an extrapolated view of the sample 110. Extrapolating is a technique used to generate additional image content beyond the boundaries of an existing image. It aims to estimate and create plausible information in areas that are not present in the original image. Thus, an extrapolated view of the sample 110 during the arrangement of the microscope can be generated. The temporary data indicative of the extrapolated view can be transmitted to a frame buffer and or a display device. In this way, a continuous image of the sample 110 can be displayed to the user, especially during arrangement of the microscope.

For example, the temporary data can be used to overcome a latency of the physical movement of the microscope. The displayed image of the sample 110 could be a calculated image, e.g., an extrapolated view of the sample 110. Optionally, the temporary data may be indicative for a temporary view of the sample at a requested arrangement of the microscope. For example, the apparatus 130 may determine a desired rotation angle of the user, e.g., a desired observation angle. Based on the desired rotation angle of the user the apparatus 130 may determine the temporary data, such that the temporary view matches the desired arrangement of the microscope, e.g., an observation angle of the microscope. In this case, the apparatus 130 may update the temporary view of the sample 110 with a live video feed as the microscope reaches the desired arrangement.

In an example, the apparatus 130 may be further configured to obtain reference point data indicative of a reference point for the arrangement of the microscope and determine the control data based on the movement data and the reference point data. For example, a reference point may be a rotation point of the microscope relative to the sample 110. The user can select a rotation point for rotating the microscope relative to the sample 110. Thus, the user can individualize the arrangement of the microscope. For example, if the user does not select a rotation point, the microscope may be rotated relative to a center point of the sample. The reference point data may be received from an input device 150. The reference point data may be determined by the apparatus 130 based on the control data. For example, the user may perform a movement with his hand to select a reference point. The apparatus 130 can determine the selected reference point of the user based on the control data.

In an example, the apparatus 130 may be further configured to receive movement sensor data of a movement sensor 124. The movement sensor data is indicative of a movement of the hand of the user. Further, the apparatus 130 may be configured to determine the control data based on the movement sensor data. For example, the apparatus 130 may receive raw data from the movement sensor 124 and may post process the movement sensor data to determine the movement of the hand of the user. Alternatively, the apparatus 130 may receive post processed movement sensor data, which can be used to determine the movement of the head of the user without further post processing. Receiving the movement sensor data from the movement sensor 124 may allow to determine the movement of the hand of the user with an increased reliability.

In an example, the apparatus 130 may be further configured to obtain verification data indicative of an intention of the user to arrange the microscope and determine the control data based on the movement data and the verification data. For example, the verification data can be used to verify an intention of the user, determined based on the movement data, to arrange the microscope. In this way, a movement of the microscope not intended by the user can be avoided.

In an example, the apparatus 130 may be further configured to determine, based on the verification data, an intention of the user to adjust an observation angle of the microscope, a magnification of the microscope and/or a field of view of the microscope. Thus, the user can adjust a desired parameter of the microscope in a facilitated way.

In an example, the apparatus 130 may be further configured to receive verification sensor data of a verification sensor 126. The verification sensor data is indicative of an intention of the user to arrange the microscope. The verification sensor 126 is different from the movement sensor 124. Further, the apparatus 130 may be configured to determine the verification data based on the verification sensor data. In an example, the verification sensor may be a microphone and/or an eye-tracking camera. For example, voice recognition and/or eye-tracking can be used in conjunction with the movement sensor data. Thus, the user can control the arrangement of the microscope using spoken commands or by his eyes (e.g., based on a gaze direction). For example, a verification of an intended change of a magnification of the microscope may be required by voice recognition. In this way, it can be ensured that an arrangement of the microscope is only adjusted according to a user's intention.

In an example, the apparatus 130 may be further configured to obtain setting data indicative of a desire of the user to change the arrangement of the microscope. Further, the apparatus may be configured to determine the control data based on the movement data and the setting data. For example, the setting data may be received from an input device 150. In this way, the user can easily select a parameter of the microscope to be adjusted.

As shown in Fig. 1 the optional one or more interfaces 132 is coupled to the respective one or more processors 134 at the apparatus 130. In examples the one or more processors 134 may be implemented using one or more processing units, one or more processing devices, any means for processing, such as a processor, a computer or a programmable hardware component being operable with accordingly adapted software. Similar, the described functions of the one or more processors 134 may as well be implemented in software, which is then executed on one or more programmable hardware components. Such hardware components may comprise a general-purpose processor, a Digital Signal Processor (DSP), a micro-controller, etc. The one or more processors 134 is capable of controlling the one or more interfaces 132, so that any data transfer that occurs over the one or more interfaces 132 and/or any interaction in which the one or more interfaces 132 may be involved may be controlled by the one or more processors 134.

In an embodiment the apparatus 130 may comprise a memory, e.g., the one or more storage devices 136 and at least one or more processors 134 operably coupled to the memory and configured to perform the method described below.

In examples the one or more interfaces 132 may correspond to any means for obtaining, receiving, transmitting or providing analog or digital signals or information, e.g., any connector, contact, pin, register, input port, output port, conductor, lane, etc. which allows providing or obtaining a signal or information. The one or more interfaces 132 may be wireless or wireline and it may be configured to communicate, e.g., transmit or receive signals, information with further internal or external components.

The apparatus 130 may be a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system.

More details and aspects are mentioned in connection with the examples described below. The example shown in Fig. 1 may comprise one or more optional additional features corresponding to one or more aspects mentioned in connection with the proposed concept or one or more examples described below (e.g., Fig. 2 - 4).

Figs. 2A-2D show different examples of an arrangement of the microscope using a visual representation of an axis.

Fig. 2A shows an example in which the users uses the visual representation of an axis 210a as virtual handle 210a for adjusting the observation angle. The virtual handle may be an axis perpendicularly to an image 212 displayed on the display device 220. For example, the virtual handle 210a may meet the image at the center. As shown in the left image of Fig. 2A the user may grab the virtual handle 210a and move its hand 216 to rotate 214a the observation angle. As can be seen in the right image the imaged sample 212 may be rotated in the same direction as indicated by the movement of the hand 216 of the user. In this way, the user can adjust the observation angle of the image 212 using the virtual handle 210a. The user experience may be a mixture of virtual reality and a computer aided design platform. The user can interact and rotate the microscope image as if it is a 3D object. In Fig. 2A the user can rotate the image sample.

Fig. 2B shows an example in which the users uses the visual representation of an axis 210b as virtual handle 210b for adjusting the observation angle. In contrast to Fig. 2A instead of rotating the imaged sample 212 the user can virtually move the microscope. The imaged sample 212 on the display device 220 is identical to the imaged sample 212 in Fig. 2A. However, the direction of rotation 214b of the hand of the user is vice versa. The user may move its hand 216 in the opposite direction in comparison to Fig. 2A. The rotation of the imaged sample 212 may be in the same direction, as the user virtually moves the microscope. In this way, the user can select a desired way to adjust the observation angle. To adjust the user and/or to ease a differentiation between both modes a visualized microscope position 218 may be displayed to the user in Fig. 2B. For example, the axis data may be indicative of the visualized microscope position.

The rotation around a center point of the imaged sample 212 is an example of a reference point used for the rotation. Figs. 2A and 2B assume the center of the imaged sample 212 to be the center of rotation of the microscope. Alternatively, the user could select the reference point, for example, by contactless clicking on the desired point before rotation. Optionally, the virtual handle 210a-210d can have any angle with respect to the imaged sample 212. The angle is not limited to a perpendicular angle. For example, different angles may be advantageous for an easier interaction with the virtual handle 210a-210d.

Fig. 2C shows an example in which the users uses the visual representation of an axis 210c for adjusting the magnification or zoom of the imaged sample 212. The user may move its hand 216 away from the display device 220. As can be seen the imaged sample 212 is in the right image smaller than in the left image of Fig. 2C. In this way, the user can adjust a magnification or zoom of the imaged sample 212. For example, the zoom of the imaged sample 212 could be adjusted using feast movements along the visual representation of the axis 210c.

Fig. 2D shows an example in which the users uses the visual representation of an axis 210d for performing a pan movement of the imaged sample 212. The user may move its hand 216 to the left relative to the display device 220. As can be seen the imaged sample 212 moves also to the left. In this way, an xy-movement of the visual representation of the axis 210d can control a pan adjustment of the microscope.

As has been described above, the apparatus may determine a temporary view of the sample to overcome a latency of the arrangement of the microscope. For example, in Fig. 2D a video feed (e.g., a continuous stream of video data that is captured by an optical imaging sensor (122 in Fig. 1) and transmitted in real-time or near real-time, e.g., by the apparatus) could be shifted left in real time, enough so that the imaged sample 212 is following the user's hand movement. As the microscope moves towards the target position, the digital shift could gradually be reduced until eventually no digital correction may be applied. Similarly, in the adjustment of the observation angle as shown in Fig. 2A and 2B, the temporary data could be indicative of a 3D-rotated captured video feed. Specifically the digital correction/pre-calculation of the observation angle could utilize the disparity of the Right-Left channels of the stereo image. This would create a more realistic impression.

More details and aspects are mentioned in connection with the examples described above and/or below. The example shown in Fig. 2 may comprise one or more optional additional features corresponding to one or more aspects mentioned in connection with the proposed concept or one or more examples described above (e.g., Fig. 1) and/or below (e.g., Fig. 3 - 4).

Fig. 3 shows a schematic illustration of a system 300, e.g., an optical imaging system 300. The optical imaging system 300 may comprise an apparatus as described with reference to Fig. 1 and a microscope 310. For example, the microscope 310 may comprise or can be communicatively coupled to the apparatus. Alternatively, the computer system 320 may comprise the apparatus. The apparatus can be used to generate control data for controlling an arrangement of the microscope 310.

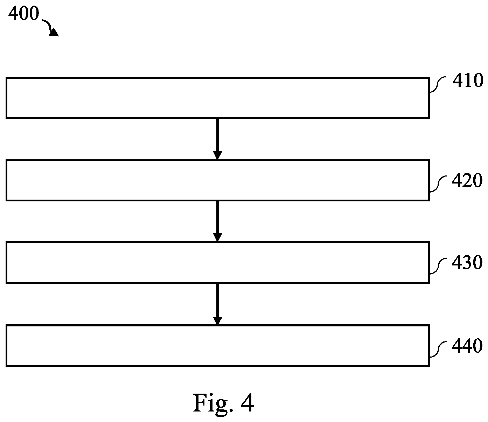

Fig. 3 shows a schematic illustration of a system 300, e.g., an optical imaging system, configured to perform a method described herein, e.g., with reference to Figs. 4. The system 300 comprises a microscope 310 and a computer system 320. The microscope 310 may comprise the apparatus as described above, e.g., with reference to Fig. 1. The microscope 310 is configured to take images and is connected to the computer system 320. The computer system 320 is configured to execute at least a part of a method described herein. The computer system 320 may be configured to execute a machine learning algorithm. The computer system 320 and the microscope 310 may be separate entities but can also be integrated together in one common housing. The computer system 320 may be part of a central processing system of the microscope 310 and/or the computer system 320 may be part of a subcomponent of the microscope 310, such as a sensor, an actor, a camera or an illumination unit, etc. of the microscope 310.

The computer system 320 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 320 may comprise any circuit or combination of circuits. In one embodiment, the computer system 320 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 320 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 320 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 320 may also include a display device, one or more speakers, and a keyboard and/or controller, which can include a mouse, trackball, touch screen, voice-recognition device, or any other device that permits a system user to input information into and receive information from the computer system 320.

More details and aspects are mentioned in connection with the examples described above and/or below. The example shown in Fig. 3 may comprise one or more optional additional features corresponding to one or more aspects mentioned in connection with the proposed concept or one or more examples described above (e.g., Fig. 1 - 2) and/or below (e.g., Fig. 4).

Fig. 4 shows a flow chart of an example of a method 400 for an optical imaging system. The method 400 comprises determining 410 axis data indicative of a visual representation of an axis for arranging a microscope of the optical imaging system. Further, the method 400 comprises 420 transmitting the axis data and obtaining 430 movement data indicative of a movement of a hand of a user of the optical imaging system relative to the visual representation of the axis. The method 400 further comprises 440 determining control data for arranging the microscope based on the movement data. The method may be performed by an apparatus as described with reference to Fig. 1.

More details and aspects are mentioned in connection with the examples described above. The example shown in Fig. 4 may comprise one or more optional additional features corresponding to one or more aspects mentioned in connection with the proposed concept or one or more examples described above (e.g., Fig. 1 - 3).

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

If some aspects have been described in relation to a device or system, these aspects should also be understood as a description of the corresponding method and vice versa. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

The aspects and features described in relation to a particular one of the previous examples may also be combined with one or more of the further examples to replace an identical or similar feature of that further example or to additionally introduce the features into the further example.

### List of reference Signs

110 sample
120 microscope
122 optical imaging sensor
124 movement sensor
126 verification sensor
130 apparatus
132 interface
134 processor
136 storage device
150 input device
210a, 210b, 210c, 210d visual representation of an axis
212 imaged sample
214a, 214b, 214c, 214d movement direction
216 hand of the user
218 visualized microscope position
300 system
310 microscope
320 computer system
400 method for an optical imaging system
410 determining axis data
420 transmitting the axis data
430 obtaining movement data
440 determining control data

## Claims

1. An apparatus (130) for an optical imaging system, comprising one or more processors (134) and one or more storage devices (136), wherein the apparatus (130) is configured to:
determine axis data indicative of a visual representation of an axis for arranging a microscope of the optical imaging system;
transmit the axis data;
obtain movement data indicative of a movement of a hand of a user of the optical imaging system relative to the visual representation of the axis; and
determine, based on the movement data, control data for arranging the microscope.

2. The apparatus (130) according to claim 1, wherein the apparatus (130) is configured to
determine, based on the movement data, gesture data indicative of a gesture being performed by the user; and
determine, based on the movement data and the gesture data, the control data.

3. The apparatus (130) according to any one of the preceding claims, wherein the apparatus (130) is further configured to
control, based on the control data, at least one of an observation angle of the microscope, a magnification of the microscope and a field of view of the microscope.

4. The apparatus (130) according to any one of the preceding claims, wherein
the visual representation of the axis is a visual representation of an optical path of the microscope or a handle of the microscope.

5. The apparatus (130) according to any one of the preceding claims, wherein the apparatus (130) is further configured to
determine temporary data indicative of a temporary view of a sample (110) for displaying during arrangement of the microscope; and
transmit the temporary data.

6. The apparatus (130) according to claim 5, wherein the apparatus (130) is further configured to
receive sensor data of a sensor (122) of the optical imaging system, the sensor data indicative of a live view of the sample (110) through the microscope of the optical imaging system; and
determine, based on the sensor data and the control data, the temporary data indicative of an extrapolated view of the sample (110).

7. The apparatus (130) according to any one of the preceding claims, wherein the apparatus (130) is further configured to
obtain reference point data indicative of a reference point for the arrangement of the microscope; and
determine, based on the movement data and the reference point data, the control data.

8. The apparatus (130) according to any one of the preceding claims, wherein the apparatus (130) is further configured to
receive movement sensor data of a movement sensor (124), the movement sensor data indicative of a movement of the hand of the user; and
determine, based on the movement sensor data, the movement data.

9. The apparatus (130) according to any one of the preceding claims, wherein the apparatus (130) is further configured to
obtain verification data indicative of an intention of the user to arrange the microscope; and
determine, based on the movement data and the verification data, the control data.

10. The apparatus (130) according to claim 9, wherein the apparatus (130) is further configured to
determine, based on the verification data, an intention of the user to adjust at least one of an observation angle of the microscope, a magnification of the microscope and a field of view of the microscope.

11. The apparatus (130) according to claim 8 and claim 9 or 10, wherein the apparatus (130) is further configured to
receive verification sensor data of a verification sensor (126), the verification sensor data indicative of an intention of the user to arrange the microscope, wherein the verification sensor (126) is different from the movement sensor (124); and
determine, based on the verification sensor data, the verification data.

12. The apparatus (130) according to any one of the preceding claims, wherein the apparatus (130) is further configured to
obtain setting data indicative of a desire of the user to change the arrangement of the microscope; and
determine, based on the movement data and the setting data, the control data.

13. An optical imaging system (300) comprising an apparatus (130) according to any one of the preceding claims.

14. Method (400), comprising:
determining (410) axis data indicative of a visual representation of an axis for arranging a microscope of the optical imaging system;
transmitting (420) the axis data;
obtaining (430) movement data indicative of a movement of a hand of a user of the optical imaging system relative to the visual representation of the axis; and
determining (440), based on the movement data, control data for arranging the microscope.

15. A computer program with a program code for performing the method according to claim 14 when the computer program is executed on a processor.
